# EUROPÄISCHE PATENTSCHRIFT

(11) **EP 3 452 168 B1**
(45) Veröffentlichungstag und Bekanntmachung des Hinweises auf die Patenterteilung: **05.08.2020**
(21) Anmeldenummer: 17713151.3
(22) Anmeldetag: 17.02.2017
(51) Int. Cl.: A61N 1/40, H05H 1/24

(54) **BEHANDLUNGSANORDNUNG UND VERFAHREN ZUR HERSTELLUNG EINER BEHANDLUNGSANORDNUNG**
TREATMENT ARRANGEMENT AND METHOD FOR PRODUCING A TREATMENT ARRANGEMENT
SYSTÈME DE TRAITEMENT ET SON PROCÉDÉ DE FABRICATION

(30) Priorität: 06.05.2016 DE 102016108450
(43) Veröffentlichungstag der Anmeldung: 13.03.2019
(73) Patentinhaber: Cinogy GmbH, 37115 Duderstadt (DE)
(72) Erfinder: HAHNL, Mirko, 37339 Berlingerode (DE); TRUTWIG, Leonhard, 37115 Duderstadt (DE); STORCK, Karl-Otto, 37115 Duderstadt (DE); WANDKE, Dirk, 37308 Heilbad Heiligenstadt (DE)
(74) Vertreter: Gramm, Lins & Partner Patent- und Rechtsanwälte PartGmbB
(86) Internationale Anmeldenummer: PCT/DE2017/100138
(87) Internationale Veröffentlichungsnummer: WO 2017/190724

(56) Entgegenhaltungen:
- WO-A1-2013/040542
- WO-A2-2012/106735
- US-A1- 2012 213 664
- US-A1- 2016 045 246

## Beschreibung

Die Erfindung betrifft eine Behandlungsanordnung zur Behandlung einer Oberfläche, mit einer flächigen Elektrodenanordnung, der eine elektrische Spannung zuführbar ist, und einer flächigen, aus einem isolierenden Kunststoff bestehenden Abschirmschicht, die die Elektrodenanordnung zumindest teilweise umgibt wobei die Elektrodenanordnung aus einem leitfähigen Zusätzen versehenen Kunststoff besteht.

Die Erfindung betrifft ferner eine Behandlungsvorrichtung mit einer derartigen Behandlungsanordnung sowie ein Verfahren zur Herstellung einer Behandlungsanordnung der erwähnten Art.

Es sind verschiedene Behandlungsanordnungen bekannt, mit denen eine Oberfläche unter der Einwirkung elektrischer Spannungen behandelt werden können. Hierzu gehören Behandlungsanordnungen, bei denen ein Strom unmittelbar in die zu behandelnde Oberfläche eingeleitet wird. Die Abschirmschicht hat dabei die Funktion, eine Berührung der Elektrode durch eine Bedienperson zu vermeiden.

Ferner ist es bekannt, mittels der Elektrode eine induktive Erwärmung der zu behandelnden Oberfläche vorzunehmen. In diesem Fall dient die Abschirmschicht der Isolierung der Elektrode gegenüber der zu behandelnden Oberfläche, um eine direkte Stromeinleitung und gegebenenfalls die Ausbildung von Überschlagsfunken zu verhindern.

In einer bevorzugten Ausführung der erfindungsgemäßen Behandlungsanordnung ist diese als Plasma-Behandlungsvorrichtung ausgebildet und dient dazu, mittels der Elektrodenanordnung ein Hochspannungsfeld aufzubauen, indem ein Luft- oder Gasraum zwischen der Behandlungsanordnung und der zu behandelnden Oberfläche zu einem Plasma ionisiert wird, sodass eine an sich bekannte Plasmabehandlung der Oberfläche erfolgt. Dabei kann im Rahmen der vorliegenden Erfindung die Elektrodenanordnung mit einem Pol einer elektrischen Spannung verbunden werden und die zu behandelnde Oberfläche eine Gegenelektrode bilden. Es ist aber auch möglich, die Elektrodenanordnung aus wenigstens zwei Elektroden zu bilden, die mit unterschiedlichen Polen einer elektrischen Spannung verbunden sind, sodass zwischen den Elektroden ein elektrisches Feld aufgebaut wird, mit dem beispielsweise Luft zur Ausbildung eines Plasmas ionisiert werden kann.

Die zu behandelnde Oberfläche kann ein Material sein, das durch die Behandlung, beispielsweise einer Plasmabehandlung, an seiner Oberfläche dazu vorbereitet wird, mit einer Schicht, beispielsweise einer Schutzschicht, versehen zu werden. Die Behandlung der Oberfläche dient dann dazu, die Haftung der Schicht nach Art einer Grundierungsbehandlung zu verbessern. Derartige Behandlungen kommen für Materialien aus Kunststoff, Metall, Holz o. dgl. in Frage.

Eine im Rahmen der Erfindung bevorzugte Anwendung ist die Behandlung der menschlichen oder tierischen Haut als zu behandelnde Oberfläche. Insbesondere für die Ausbildung eines Plasmas sind vorteilhafte Effekte auf die Hautoberfläche nachgewiesen worden. Insbesondere hat die Plasmabehandlung eine desinfizierende, also keimtötende Wirkung. Es ist daher bekannt, eine Behandlungsanordnung für eine Plasmabehandlung in Form einer Wundauflage auszubilden. Die der Wunde zugekehrte Schicht der Behandlungsanordnung kann dabei aus Silikon gebildet sein, das bekanntlich hautfreundlich und hautverträglich ist und als isolierenden Schicht geeignet ist. Ein in Frage kommendes Material ist beispielsweise das Silikongel SILPURAN® der Wacker Chemie AG, Burghausen, Deutschland. Dieses Zwei-Komponenten-Silikongel vernetzt zu einer weichen Silikonschicht, die eine gewisse Klebrigkeit aufweist und daher auf der Haut haftet.

In Frage kommende Formen derartiger Behandlungsanordnungen sind beispielsweise durch DE 10 2014 013 716 A1 bekannt und für die Behandlung der Haut beziehungsweise von Wunden mit einem dielektrisch behinderten Plasma bekannt. Dabei ist eine metallische Elektrode vollständig in einem Dielektrikum, das ein Silikon sein kann, eingebettet. Um den Abfluss von Wundsekret zu ermöglichen, kann das Dielektrikum mit Durchgangsöffnungen versehen sein, durch die Wundsekret von der Wundseite der Behandlungsanordnung auf die distale Seite hindurch treten kann, wo es beispielsweise von einem Absorptionsmaterial aufgenommen werden kann. Die eingebettete Elektrode muss dabei mit entsprechenden Durchgangsöffnungen versehen sein, deren Durchmesser allerdings größer ist als der Durchmesser der Durchgangsöffnungen in dem Dielektrikum, sodass das Dielektrikum die Elektrode auch im Bereich der durch die Durchgangsöffnungen gebildeten Kanäle zuverlässig abdeckt. Die in dem Dielektrikum eingebettete Elektrode ist dabei flächig und flexibel einpolig ausgebildet, sodass der zu der Hautoberfläche gehörende Körper als Gegenelektrode fungiert. Die Gegenelektrode kann dabei geerdet sein oder als "floatende Elektrode" wirken. Der Aufbau der bekannten Behandlungsanordnungen hat sich bewährt, da sowohl das Dielektrikum als auch die Elektrode selbst flexibel ausgebildet werden können und somit die gesamte Behandlungsanordnung dafür geeignet ist, sich der gegebenenfalls unregelmäßigen Form eines Körperteils anzupassen und so die Behandlung einer unversehrten Hautoberfläche oder einer Wunde mit definierten Abstandsverhältnissen und somit reproduzierbaren Ergebnissen durchzuführen.

US 2012/0213664 A1 offenbart eine Behandlungsanordnung für eine Plasmabehandlung, mit der als Folienbeutel oder Flasche ausgebildete Behälter für Lebensmittel o.dgl. behandelt werden können. Die Behandlungsanordnung weist zwei Elektroden auf, wobei die dem zu behandelnden Artikel zugewandte Elektrode als flächige Masseelektrode Unterbrechungen aufweist, durch die sich das Plasmafeld zur Erzeugung eines Oberflächenplasmas erstrecken kann. Zwischen den Elektroden befindet sich ein flexibles Dielektrikum. In einem Ausführungsbeispiel bildet das Dielektrikum mit der Hochspannungselektrode auf einer Innenseite und der unterbrochenen Masseelektrode auf der Außenseite eine flexible Wandung, die sich an die Form eines Gegenstands, beispielsweise einer Flasche, anpassen kann. Hierzu ist die Wandung über einen Hohlraum mit einem Grundkörper des Dielektrikums verbunden. Die auf die Oberflächen der flexiblen Wandung aufgebrachten Elektroden können aus einem leitenden Silikonpolymer bestehen. Über die Verbindung der Elektroden mit der dielektrischen Wandung ist nichts offenbart. Die Behandlungsanordnung ist ersichtlich nicht für die Behandlung der menschlischen oder tierischen Haut oder von Wundflächen vorgesehen.

WO 2012/106735 A2 offenbart verschiedene Ausbildungen von Behandlungsanordnungen, bei denen eine leitende Elektrode in einem flächigen flexiblen Dielektrikum eingebettet ist. Für das Ausführungsbeispiel einer Gesichtsmaske wird vorgeschlagen, eine erste Dielektrikumschicht in einer dem Gesicht entsprechenden Negativform auszubilden, dort eine leitende Elektrodenschicht aufzubringen, die mit einer weiteren dielektrischen Schicht eingeschlossen wird. Beispiele für die elektrisch leitende Schicht ist eine leitende Tinte, eine Metallfolie oder ein leitendes Gel. Eine eigene Verbindung der leitenden Schicht mit dem Dielektrikum ist nicht offenbart.

Der vorliegenden Erfindung liegt die Aufgabe zugrunde, den Aufbau einer Behandlungsanordnung der eingangs erwähnten Art zu vereinfachen und noch zuverlässiger zu gestalten.

Zur Lösung dieser Aufgabe ist erfindungsgemäß eine Behandlungsanordnung der eingangs erwähnten Art dadurch gekennzeichnet, dass im Bereich der Grenzschicht zwischen Elektrodenanordnung und Abschirmschicht die Kunststoffe der Elektrodenanordnung und der Abschirmschicht ohne eine zusätzliche Klebschicht materialschlüssig dadurch miteinander verbunden sind, dass die Kunststoffe im Bereich der Grenzschicht miteinander gemischt und/oder miteinander vernetzt sind.

Bei der erfindungsgemäßen Behandlungsanordnung wird somit eine Elektrode nicht als metallische Elektrode mit der dielektrischen Kunststoffschicht zumindest teilweise umgeben, insbesondere vollständig eingebettet, sondern die Elektrode wird selbst aus einem geeigneten Kunststoff ausgebildet, der durch Zusätze leitfähig gemacht wird. Derartige Elektroden sind an sich bekannt. Erfindungsgemäß werden sie zur Ausbildung der Behandlungsanordnung eingesetzt, um so eine materialschlüssige Verbindung zwischen der Elektrodenanordnung und der Abschirmschicht zu ermöglichen, die sich durch die Kunststoffe selbst ergibt und keine zusätzliche Klebschicht an der Grenzschicht zwischen der Elektrodenanordnung und der Abschirmschicht aufweist. Elektrodenanordnung und Abschirmschicht können somit quasi als einheitliches Material ausgebildet werden und gewähren dadurch eine hohe Sicherheit gegen eine Delaminierung der Elektrodenanordnung von der Abschirmschicht. Dies ist insbesondere von Bedeutung, wenn die Behandlungsanordnung in vorteilhafter Weise flexibel ist und starke Biegungen ermöglicht, um sich auch schwierigen Körperteilen anpassen zu können, also beispielsweise ein Handgelenk umschlingen können.

Die erfindungsgemäße materialschlüssige Verbindung zwischen dem Kunststoff der Elektrodenanordnung und dem Kunststoff der Abschirmschicht gelingt nach einer Mischung dieser Kunststoffe zumindest im Bereich der Grenzschicht in einfacher Weise, wenn die Kunststoffe - zumindest in diesem Bereich - gemeinsam aushärten und/oder vernetzen. In einer weiter bevorzugten Ausführungsform werden für die Elektrodenanordnung und die Abschirmschicht chemisch gleiche Kunststoffe verwendet, die sich dadurch gut mischen lassen. Für die Elektrodenanordnung ist der Kunststoff dabei lediglich mit den leitfähigen Zusätzen versehen, die metallische Teilchen, beispielsweise Mikro- oder Nanoteilchen, Graphitpulver o. ä. sein können.

Alternativ ist es möglich, auf die Mischung der Kunststoffe zu verzichten und die materialschlüssige Verbindung dadurch herzustellen, dass die in der Grenzschicht aneinanderstoßenden Kunststoffe von Elektrodenanordnung und Abschirmschicht miteinander vernetzen. Dies kann dadurch geschehen, dass ein erster der Kunststoffe zunächst nur teilvernetzt wird und mit dem zweiten der Kunststoffe bei dessen Vernetzung im Sinne der ursprünglichen Vernetzung weitervernetzt wird. In einer anderen Ausführungsform kann der erste der Kunststoffe auch vollständig vernetzt werden, wenn er funktionelle vernetzbare randständige Gruppen aufweist, die bei Zuführung des unvernetzten zweiten der Kunststoffe bei dessen Vernetzung zu einer Sekundärvernetzung zwischen dem ersten Kunststoff und dem zweiten Kunststoff führen.

In einer bevorzugten Ausführungsform der Erfindung ist die Elektrodenanordnung allseitig von der Abschirmschicht umschlossen. Dies ist insbesondere vorteilhaft, wenn der Elektrode eine Hochspannung zugeführt wird, wie dies für die Behandlung mit einem dielektrisch behinderten Plasma in einer bevorzugten Ausführungsform der Erfindung der Fall ist.

In dieser Konfiguration ist es möglich, einen elektrisch leitenden Anschluss der Elektrodenanordnung aus der Abschirmschicht herauszuführen. Alternativ ist es möglich, die Elektrodenanordnung beispielsweise mit einer Anschlusszunge, die ebenfalls von der Abschirmschicht umschlossen ist, auszubilden und die elektrische Spannung mit einer Kontaktanordnung zuzuführen, die die Abschirmschicht durchstößt und dadurch den Kontakt zur Elektrodenanordnung herstellt. Eine derartige Kontaktierung mit einem selbstschneidenden Kontakt ist beispielsweise durch EP 2 723 447 B1 bekannt.

Auch für die erfindungsgemäße Behandlungsanordnung kann es zweckmäßig sein, wenn die Abschirmschicht auf der Behandlungsseite zur Ausbildung von Anlageflächen profiliert ist, zwischen denen bei Anlage der Behandlungsanordnung an der zu behandelnden Oberfläche die Luftzwischenräume zur Ausbildung des Plasmas bestehen. Dabei kann die Profilierung unregelmäßig oder regelmäßig sein. Aus EP 2 515 997 A1 ist eine Profilierung in Form von runden Noppen bekannt, aus DE 10 2013 019 057 A1 auch in Form von einseitig offenen Kammern, die auf der Behandlungsseite der Abschirmschicht ausgebildet sind und gegebenenfalls mit pflegenden oder heilenden Substanzen gefüllt werden können.

Für die Anwendung als Wundbehandlungsanordnung ist es zweckmäßig, wenn auf der Behandlungsseite eine Wundauflagenfläche ausgebildet ist. Diese kann durch das geeignete Material der Abschirmschicht selbst gebildet sein oder auf die Behandlungsseite der Abschirmschicht ergänzend aufgebracht sein.

Für die Abschirmschicht und die Elektrodenanordnung eignen sich insbesondere Silikone in jeder Form, bevorzugt in Form von Silikongelen.

Insbesondere für den Zweck der Wundversorgung eignet sich eine Ausführungsform der erfindungsgemäßen Behandlungsanordnung, bei der die Abschirmschicht über die Fläche der Elektrode hinausragende Abschnitte aufweist, die zur zu behandelnden Oberfläche hin klebend ausgebildet sind. Gegebenenfalls kann hierfür die klebende Eigenschaft eines Silikongels selbst ausgenutzt werden. In diesem Fall kann die Silikonschicht bereits die Befestigung der gesamten Anordnung auf der Haut in der Umgebung der Wunde bewirken, sodass gegebenenfalls auf einen Sekundärverband zusätzlich zu der Behandlungsanordnung verzichtet werden kann.

Als Elektrodenanordnung einer erfindungsgemäßen Behandlungsanordnung kann eine einpolige Elektrode verwendet werden, wenn die zu behandelnde Fläche beziehungsweise der dahinter befindliche Körper als Gegenelektrode fungiert. Alternativ kann die Elektrodenanordnung wenigstens zweipolig ausgebildet sein, wobei die beiden Pole mit den Polen einer Spannungsversorgung verbunden werden. Die Elektroden sind dann zweckmäßigerweise so geformt und positioniert, dass über die Fläche der Behandlungsanordnung in vielen Bereichen die Elektrode des einen Pols nahe und parallel zu der Elektrode des anderen Pols verläuft, sodass zwischen diesen beiden Polen ein für die Plasmabildung geeignetes räumliches elektrisches Feld entsteht. Damit das elektrische Feld über die Fläche verteilt und nicht nur lokal vorhanden ist, werden die beiden Elektrodenpole vorzugsweise streifenförmig ausgebildet und über die Fläche der Behandlungsanordnung parallel oder antiparallel geführt. Hierbei eignen sich Mäanderformen, spiralförmige Verläufe, kammförmige Strukturen usw.

Mit einer erfindungsgemäßen Behandlungsanordnung wird bevorzugt eine Behandlungsvorrichtung gebildet, die zur Veränderung eines direkten Kontakts der Elektrodenanordnung und der zu behandelnden Oberfläche ausgebildet und positioniert ist. Wie bereits erläutert, kann es sich insbesondere für eine dielektrisch behinderte Plasmabehandlung anbieten, die Elektrodenanordnung vollständig in der elektrischen Abschirmschicht einzubetten.

Die Herstellung des erfindungsgemäßen Materialschlusses zwischen dem Kunststoff der Elektrodenanordnung und dem Kunststoff der Abschirmschicht gelingt dadurch, dass zumindest im Bereich einer Grenzfläche zwischen der Elektrodenanordnung und der Abschirmschicht die Kunststoffe in einem flüssigen Zustand miteinander vermischt und gemeinsam ausgehärtet und/oder vernetzt werden. Auf diese Weise wird der Materialschluss durch eine einheitliche Matrixstruktur oder einen allmählichen Übergang von einer Matrixstruktur auf die andere Matrixstruktur bewirkt.

Es ist durchaus möglich, die Elektrodenanordnung und/oder die Abschirmschicht vorzufertigen und teilweise oder ganz zu vernetzen oder auszuhärten und anschließend im Bereich einer Grenzfläche zu verflüssigen oder zumindest aufzuquellen, um so eine gemeinsame Aushärtung und/oder Vernetzung in diesem Bereich zu erreichen.

Bevorzugt ist jedoch, dass in eine Gießform zunächst wenigstens eine Lage der Abschirmschicht und dann der mit den leitfähigen Zusätzen versehende Kunststoff der Elektrodenanordnung, jeweils in flüssigem Zustand, eingebracht wird, sodass sich im Grenzbereich zwischen den Kunststoffen eine Vermischung ergibt, und dass anschließend die Kunststoffe gemeinsam durch Abkühlung ausgehärtet und/oder vernetzt werden. Dabei kann die etwaige Vernetzung, beispielsweise bei einem Silikongel, durch eine Vernetzungskomponente erfolgen, die temperaturabhängig oder temperaturunabhängig zur Vernetzung führt.

Auf die Vermischung der Kunststoffe im Grenzbereich kann im Falle der Vernetzung der Kunststoffe miteinander verzichtet werden. Hierfür kann vorgesehen werden, dass die Elektrodenanordnung oder die Abschirmschicht zunächst mit dem Kunststoff geformt und teilvernetzt wird. In einer Form, beispielsweise einer Spritzgussform, kann dann der andere Kunststoff eingespritzt und zur Vernetzung gebracht werden, wobei der zweite Kunststoff so gewählt ist, dass dabei der erste, zunächst nur teilvernetzte Kunststoff weitervernetzt wird.

Alternativ hierzu ist es möglich, den ersten Kunststoff praktisch vollständig zu vernetzen und dabei mit funktionellen, vernetzbaren Gruppen zu versehen. Dabei kann der erste Kunststoff als vorgeformtes Teil in eine Spritzgussform eingelegt werden, mit der die Formung des zweiten Kunststoffs erfolgt, der im unvernetzten flüssigen Zustand zugeführt wird. Der zweite Kunststoff wird dabei so gewählt, dass er mit den vernetzbaren Gruppen des ersten Kunststoffteils eine Sekundärvernetzung herstellt. Insbesondere bei Silikonen ist dies durch OH-Gruppen möglich, die eine Polykondensationsreaktion unter Abspaltung von Wasser erlauben. Ein anderes Beispiel ergibt sich mit restlichen reaktiven SiH-Gruppen, die eine additive Vernetzung mit reaktiven Vinylgruppen des anderen Silikonkunststoffes ermöglichen. Für die Sekundärvernetzung sind aber auch alle anderen Polymerisationsreaktionen geeignet.

Die Erfindung soll im Folgenden anhand von in der Zeichnung dargestellten Ausführungsbeispielen näher erläutert werden. Es zeigen:
- Figur 1 -: eine Draufsicht auf ein erstes Ausführungsbeispiel einer erfindungsgemäßen Behandlungsanordnung, die an eine Spannungsversorgungseinrichtung angeschlossen ist;
- Figur 2 -: einen Schnitt durch die Behandlungsanordnung gemäß Figur 1 entlang der Linie A-A;
- Figur 3 -: das Ausführungsbeispiel gemäß Figur 1 einer Behandlungsanordnung, die an eine modifizierte Spannungsversorgungseinrichtung angeschlossen ist;
- Figur 4 -: die Anordnung gemäß Figur 3 in einer Darstellung zur Verdeutlichung der Elektrodenanordnung;
- Figur 5 -: eine Draufsicht auf ein zweites Ausführungsbeispiel einer erfindungsgemäßen Behandlungsanordnung;
- Figur 6 -: einen Schnitt durch die Behandlungsanordnung gemäß Figur 5 entlang der Linie B-B;
- Figur 7 -: eine Draufsicht auf eine dritte Ausführungsform einer erfindungsgemäßen Behandlungsanordnung, die an einen Pol einer Hochspannungsversorgungseinrichtung angeschlossen ist;
- Figur 8 -: einen Schnitt durch die Behandlungsanordnung gemäß Figur 7;
- Figur 9 -: eine Ansicht von unten auf die Behandlungsanordnung gemäß Figur 7.

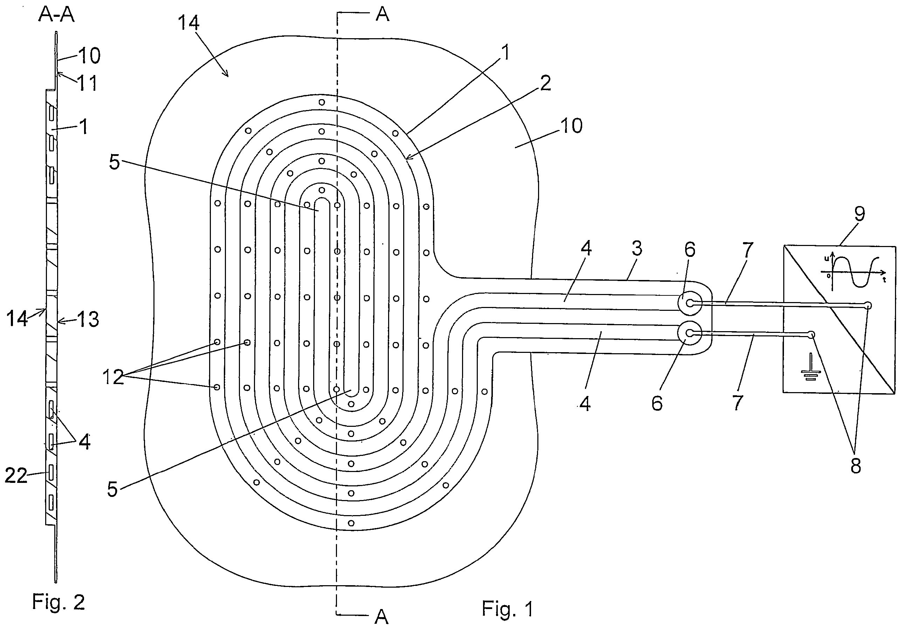

Die Figuren 1 und 2 illustrieren eine Behandlungsanordnung, in der eine dielektrische Abschirmschicht 1, in die eine Elektrodenanordnung 2 so eingebettet ist, dass die Abschirmschicht 1 die Elektrodenanordnung 2 allseitig umgibt. Hierzu ist die Abschirmschicht 1 mit einer solchen Dicke ausgebildet, dass die Elektrodenanordnung 2 allseitig mit einer ausreichend dicken dielektrischen Abschirmung umgeben ist, die einen merkbaren Stromfluss verhindert. Die Abschirmschicht 1 bildet eine seitliche Anschlusszunge 3 aus, in die sich die Elektrodenanordnung 2 erstreckt.

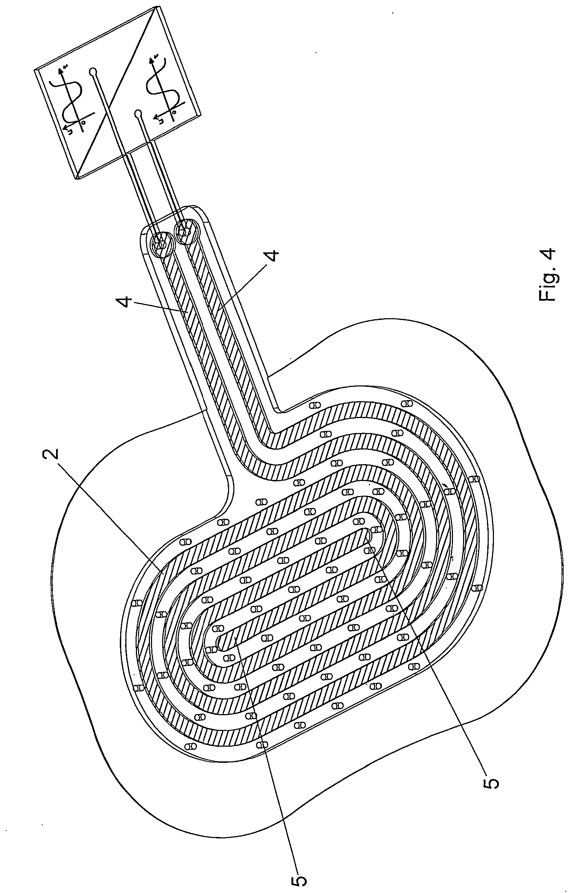

Wie insbesondere Figur 4 erkennen lässt, weist die Elektrodenanordnung 2 zwei Elektrodenstreifen 4 auf, die als streifenförmige Leiter parallel zueinander verlaufen und in einer ovalen Form spiralförmig gewunden sind, wobei innere Enden 5 in antiparallel zeigenden geraden Stücken in jeweils einer Schleife des anderen Elektrodenstreifens abgeschlossen sind. In der Anschlusszunge 3 verlaufen die beiden Elektrodenstreifen parallel zueinander und enden in Kontaktflächen 6, die über Verbindungsleitungen 7 mit jeweils einem Pol 8 einer Hochspannungsversorgungseinrichtung 9 verbunden sind. In Figur 1 ist schematisch verdeutlicht, dass an einem Pol eine Wechselspannung anliegt, die um ein Massepotential herum oszilliert, während der andere Pol 8 auf dem Massepotential liegt. Die Elektrodenanordnung 2 wird somit mit einer alternierenden Wechsel-Hochspannung versorgt. Die beiden Elektrodenstreifen 4 sind so angeordnet, dass sie immer mit parallel verlaufenden Abschnitten miteinander abwechseln, sodass die Wechsel-hochspannung der Hochspannungsversorgungseinrichtung 9 immer zwischen den parallel zueinander liegenden Abschnitten der Elektrodenstreifen anliegt und dort lokale elektrische Felder erzeugen, die zur Ausbildung eines dielektrisch behinderten Plasmas geeignet sind.

Die dielektrische Abschirmschicht 1 ist einstückig mit Abschnitten 10 versehen, die sich allseitig - mit Ausnahme der Anschlusszunge 3 - über die Elektrodenanordnung 2 und die die Elektrodenanordnung 2 einbettende Abschirmschicht 1 erstrecken und an ihrer Unterseite 11 klebend ausgebildet sind, sodass die Behandlungsanordnung mit den an der Unterseite 11 klebenden Abschnitten 10 auf der Haut eines Körperteils nach Art eines Pflasters befestigt werden kann.

Figur 2 verdeutlicht die geringere Dicke der Abschnitte 10 gegenüber der übrigen Abschirmschicht 1, die die Elektrodenanordnung 2 in Form der Elektrodenstreifen 4 allseitig einbettet.

Die Figuren 1 und 2 lassen noch erkennen, dass die dielektrische Abschirmschicht 1 außerhalb der Elektrodenstreifen 4 mit Durchgangsöffnungen 12 versehen ist, über die einerseits Luft an eine Wundfläche gelangen kann und andererseits von einer Wundfläche Wundsekret von der eine Behandlungsseite 13 bildenden Unterseite der Abschirmschicht 1 auf die distal liegende Oberseite 14 transportierbar ist.

Wie Figur 1 verdeutlicht, befinden sich die Durchgangsöffnungen in den Zwischenräumen zwischen den Elektrodenstreifen 4, sodass die Isolation der Elektrodenanordnung 2 durch die Durchgangsöffnungen nicht gefährdet ist.

Figur 2 lässt darüber hinaus erkennen, dass die Elektrodenanordnung 2 eine flächige Anordnung mit einer geringen Höhenerstreckung ist, die sich in dieser Ausführungsform der Erfindung durch die flachen Elektrodenstreifen bildet. Diese sind vorzugsweise aus einem durch leitende Zusätze elektrisch leitfähigen Silikon gebildet, das dem Silikon entspricht, aus dem die dielektrische Abschirmschicht 1 besteht.

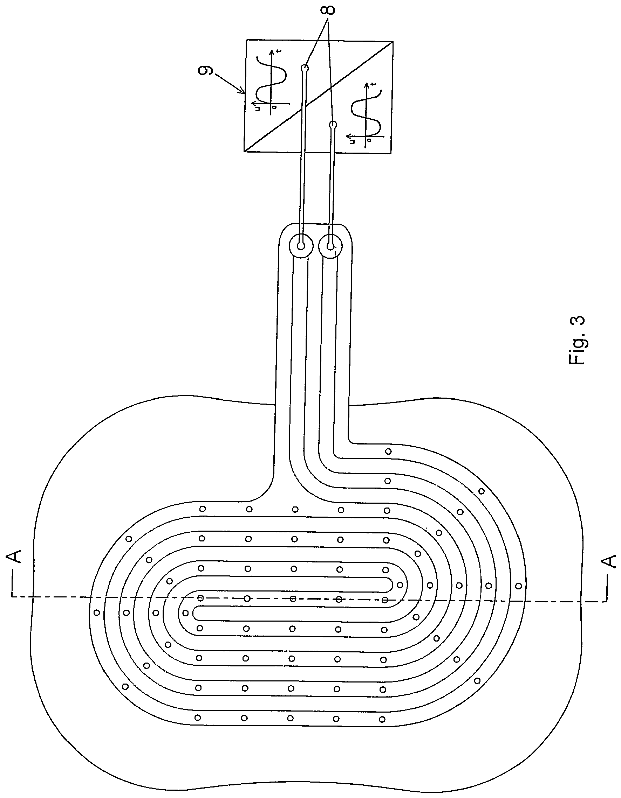

Figur 3 verdeutlicht lediglich, dass die beiden Pole 8 der Hochspannungsversorgungseinrichtung beide an alternierende Wechselspannungen angeschlossen sein können, die zueinander eine Phasenverschiebung von 180 ° aufweisen, sodass die resultierende Spannungsdifferenz zur Ausbildung der lokalen elektrischen Felder zwischen den Elektrodenstreifen 4 eine doppelte Amplitude aufweisen.

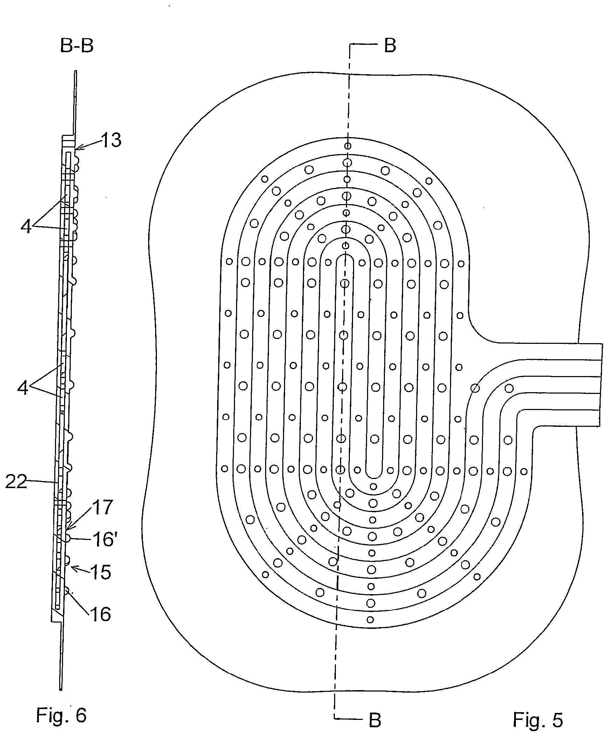

Das in den Figuren 5 und 6 dargestellte zweite Ausführungsbeispiel unterscheidet sich von dem ersten Ausführungsbeispiel gemäß den Figuren 1 bis 4 lediglich dadurch, dass die Behandlungsseite 13 der dielektrischen Abschirmschicht 1 nicht glatt ausgebildet ist, sondern eine Profilierung 15 in Form von halbkugelförmigen Erhebungen aufweist, mit deren Oberseiten die Behandlungsanordnung auf der zu behandelnden Oberfläche, also insbesondere auf der Haut eines Körperteils, aufliegen kann. Zwischen den Auflageflächen 16' befinden sich Luftzwischenräume 17, in denen sich ein Plasma durch die zwischen den Elektrodenstreifen 4 aufgebauten elektrischen Felder bilden kann, wenn die Behandlungsanordnung auf der Haut eines Körperteils aufliegt.

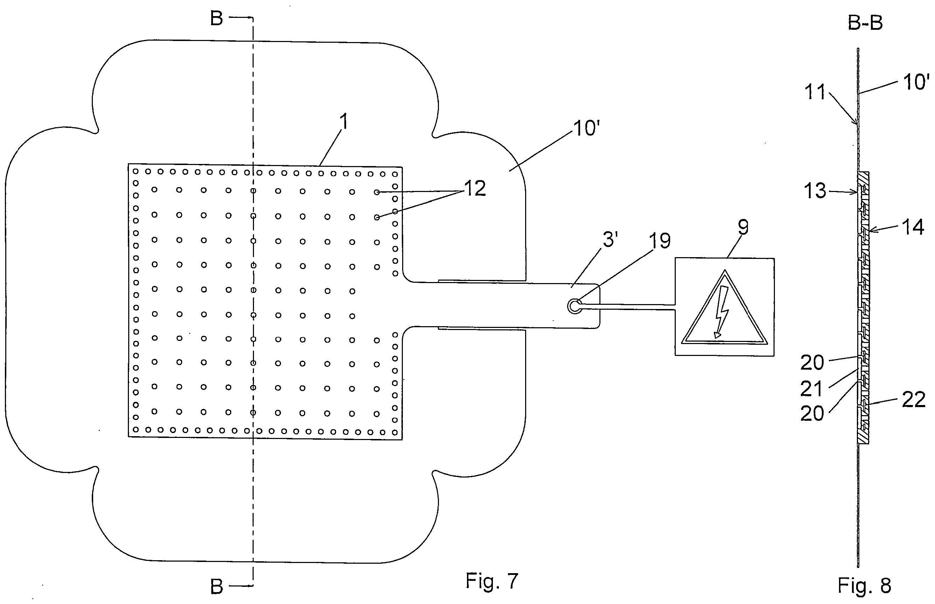

Das in den Figuren 7 bis 9 dargestellte Ausführungsbeispiel weist eine im Wesentlichen quadratische Fläche der dielektrischen Abschirmschicht 1 auf, an die sich einstückig Abschnitte 10' kleeblattartig anschließen. Die Elektrodenanordnung 2' ist durch eine durchgehend elektrisch leitende Fläche gebildet, in der sich kreisförmige Durchgangsöffnungen 18 befinden. Die elektrisch leitende Fläche der Elektrodenanordnung 2' ist allseitig in die dielektrische Abschirmschicht 1 eingebettet. Konzentrisch mit den Durchgangsöffnungen 18 verlaufen Durchgangsöffnungen 12 der dielektrischen Abschirmschicht, deren Durchmesser jedoch deutlich kleiner ist als der Durchmesser der Durchgangsöffnungen 18 in der Elektrodenanordnung 2'. Dadurch ist sichergestellt, dass auch im Bereich der Durchgangsöffnungen 12, die für eine Belüftung der Wundfläche und einen Abtransport von Wundsekret sorgen, immer eine ausreichende Isolierung zur Elektrodenanordnung 2' vorhanden ist. Auch die dielektrische Abschirmschicht 1 gemäß diesem Ausführungsbeispiel weist eine Anschlusszunge 3' auf, in die sich ein entsprechender Ansatz der Elektrodenanordnung 2' hinein erstreckt, wobei auch im Bereich der Anschlusszunge 3' die Elektrodenanordnung 2' vollständig von der dielektrischen Abschirmschicht 1 allseitig abgeschirmt ist. Eine Kontaktierung erfolgt über einen Kontaktpunkt 19, über den ein Hochspannungspotential der Hochspannungsversorgungseinrichtung 9 auf die Elektrodenanordnung 2' geleitet wird. In dieser Ausführungsform bildet der Körper der zu behandelnden Oberfläche eine Gegenelektrode für die Wechsel-Hochspannung der Hochspannungsversorgungseinrichtung 9.

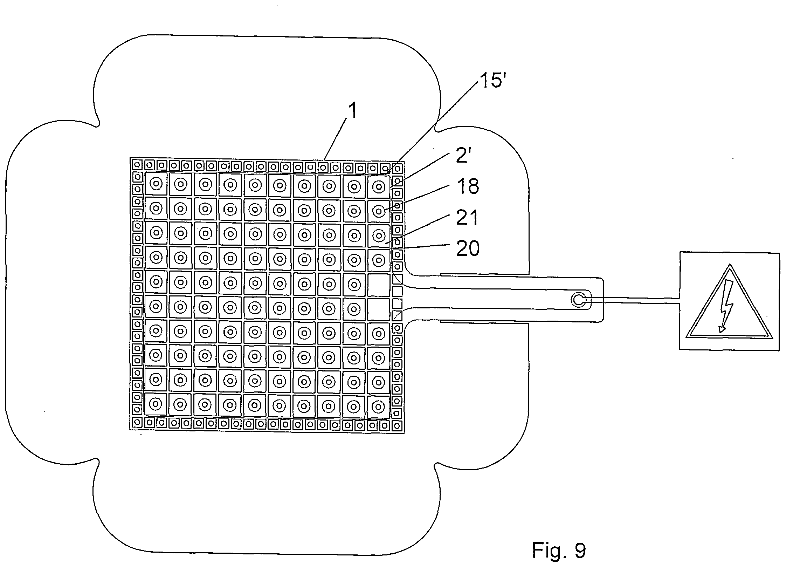

Die Ansicht von unten gemäß Figur 9 verdeutlicht eine Profilierung 15' der Behandlungsseite 13 der dielektrischen Abschirmschicht 1. Die Profilierung 15' ist mit gitterförmig ausgerichteten Wänden 20 gebildet, die um die Durchgangsöffnungen 12, 18 herum zur zu behandelnden Oberfläche offene Kammern 21 (Figur 8) bilden, in denen sich, wie in den Luftzwischenräumen 17 der vorhergehenden Ausführungsformen, ein Plasma ausbilden kann, wenn die Behandlungsanordnung auf der Haut bzw. Wundfläche eines Körpers aufliegt.

Wie die Figuren 6 und 8 verdeutlichen, besteht zwischen den Elektrodenanordnungen 2, 2' und den sie umgebenden dielektrischen Abschirmschichten 1 jeweils eine Grenzschicht 22, über die erfindungsgemäß die Materialien der Elektrodenanordnungen 2, 2' und der Abschirmschichten 1 materialschlüssig miteinander verbunden sind. Es kann vorteilhaft sein, wenn die Elektrodenanordnungen 2, 2' und die Abschirmschichten 1 aus chemisch im Wesentlichen gleichen Kunststoffen bestehen, wie sogenannte liquid silicone rubbers oder Silikongele. Diese Kunststoffmaterialien sind als Kunststoffmatrix isolierend. Für die Elektrodenanordnungen 2, 2' werden dem isolierenden Kunststoffmaterial leitfähige Zusatzstoffe beigemischt, sodass die benötigte leitfähige Ausbildung der Elektrodenanordnungen 2, 2' trotz der Verwendung der isolierenden Kunststoffmatrix ermöglicht wird. Auf diese Weise gelingt eine gegen Delaminierung auch bei starken Verformungen der flexiblen Behandlungsanordnung sichere Verbindung zwischen der Elektrodenanordnung 2, 2' und der dielektrischen Abschirmschicht 1.

Allerdings ist es auch möglich, im Rahmen der Erfindung verschiedene Kunststoffe für die Elektrodenanordnungen 2, 2' und die Abschirmschichten 1 zu verwenden, die sich entweder unmittelbar miteinander oder über eine Sekundärvernetzung im Bereich der Grenzschicht 22 miteinander vernetzen lassen.

## Patentansprüche

1. Behandlungsanordnung zur Behandlung einer Oberfläche, mit einer flächigen Elektrodenanordnung (2, 2'), der eine elektrische Spannung zuführbar ist, und einer flächigen, aus einem isolierenden Kunststoff bestehenden Abschirmschicht (1), die die Elektrodenanordnung (2, 2') zumindest teilweise umgibt, wobei die Elektrodenanordnung (2, 2') aus einem mit leitfähigen Zusätzen versehenen Kunststoff besteht, **dadurch gekennzeichnet, dass** im Bereich der Grenzschicht (22) zwischen Elektrodenanordnung (2, 2') und Abschirmschicht (1) die Kunststoffe der Elektrodenanordnung (2, 2') und der Abschirmschicht (1) ohne eine zusätzliche Klebschicht materialschlüssig dadurch miteinander verbunden sind, dass die Kunststoffe im Bereich der Grenzschicht (22) miteinander gemischt und/oder miteinander vernetzt sind.

2. Behandlungsanordnung nach Anspruch 1, **dadurch gekennzeichnet, dass** die Elektrodenanordnung (2, 2') allseitig von der Abschirmschicht (1) umschlossen ist.

3. Behandlungsanordnung nach Anspruch 1 oder 2, **dadurch gekennzeichnet, dass** ein elektrisch leitender Anschluss der Elektrodenanordnung (2, 2') aus der Abschirmschicht (1) herausgeführt ist.

4. Behandlungsanordnung nach einem der Ansprüche 1 bis 3, **dadurch gekennzeichnet, dass** eine Kontaktanordnung zur Zuführung der elektrischen Spannung durch die Abschirmschicht (1) zur Elektrodenanordnung (2, 2') hindurchführbar ist.

5. Behandlungsanordnung nach einem der Ansprüche 1 bis 4, **dadurch gekennzeichnet, dass** die Abschirmschicht (1) auf der Behandlungsseite (13) zur Ausbildung von Anlageflächen (16') profiliert ist, zwischen denen bei Anlage der Behandlungsanordnung an der zu behandelnden Oberfläche Luftzwischenräume (17) zur Ausbildung des Plasmas bestehen.

6. Behandlungsanordnung nach einem der Ansprüche 1 bis 5, **gekennzeichnet durch** eine auf der Behandlungsseite (13) ausgebildete Wundauflagenfläche.

7. Behandlungsanordnung nach einem der Ansprüche 1 bis 6, **dadurch gekennzeichnet, dass** die Kunststoffe der Elektrodenanordnung (2, 2') und der Abschirmschicht (1) chemisch identisch sind.

8. Behandlungsanordnung nach einem der Ansprüche 1 bis 7, **dadurch gekennzeichnet, dass** die Kunststoffe der Elektrodenanordnung (2, 2') und der Abschirmschicht (1) Silikone sind.

9. Behandlungsanordnung nach einem der Ansprüche 1 bis 8, **dadurch gekennzeichnet, dass** die Abschirmschicht (1) über die Fläche der Elektrodenanordnung (2, 2') herausragende Abschnitte (10) aufweist, die zur zu behandelnden Oberfläche hin klebend ausgebildet sind.

10. Behandlungsanordnung nach einem der Ansprüche 1 bis 9, **dadurch gekennzeichnet, dass** die Elektrodenanordnung (2, 2') mit einem Pol der elektrischen Spannung verbindbar und so ausgebildet ist, dass die zu behandelnde Oberfläche eine Gegenelektrode bilden kann.

11. Behandlungsanordnung nach einem der Ansprüche 1 bis 10, **dadurch gekennzeichnet, dass** die Elektrodenanordnung (2) zwei Elektrodenstreifen (4) aufweist, die mit zwei spannungsführenden Polen (8) der elektrischen Spannung verbindbar sind.

12. Behandlungsvorrichtung mit einer Behandlungsanordnung nach einem der Ansprüche 1 bis 11, **gekennzeichnet durch** eine Hochspannungsversorgungseinrichtung (9), die mit der Elektrodenanordnung (2, 2') zur Ausbildung eines Plasmas zwischen einer flächigen Behandlungsseite (13) der Behandlungsanordnung und der zu behandelnden Oberfläche verbunden ist.

13. Verfahren zur Herstellung einer Behandlungsanordnung nach einem der Ansprüche 1 bis 12, **dadurch gekennzeichnet, dass** zumindest im Bereich einer Grenzfläche (22) zwischen Elektrodenanordnung (2, 2') und Abschirmschicht (1) die Kunststoffe in einem flüssigen Zustand miteinander vermischt und gemeinsam ausgehärtet und/oder vernetzt werden.

14. Verfahren nach Anspruch 13, **dadurch gekennzeichnet, dass** in eine Gießform zunächst wenigstens eine Lage der Abschirmschicht (1), dann mit den leitfähigen Zusätzen versehener Kunststoff der Elektrodenanordnung (2, 2') im flüssigen Zustand eingebracht wird, sodass sich im Grenzbereich (22) zwischen den Kunststoffen eine Vermischung ergibt und dass anschließend die Kunststoffe gemeinsam ausgehärtet und/oder vernetzt werden.

15. Verfahren nach Anspruch 13, **dadurch gekennzeichnet, dass** ein erster der Kunststoffe teilvernetzt wird und dass anschließend ein zweiter der Kunststoffe auf den teilvernetzten ersten Kunststoff im unvernetzten Zustand geleitet wird und mit der Vernetzung des zweiten Kunststoffs eine weitere Vernetzung des teilvernetzten ersten Kunststoffs durchgeführt wird.

16. Verfahren nach Anspruch 13, **dadurch gekennzeichnet, dass** ein erster der Kunststoffe im vernetzten Zustand vernetzbare funktionelle Gruppen aufweist und dass mit einem zweiten der Kunststoffe eine materialschlüssige Verbindung durch eine Sekundärvernetzung des zweiten Kunststoffs mit den vernetzbaren Gruppen des ersten Kunststoffs hergestellt wird.

## Claims

1. Treatment arrangement for the treatment of a surface, with a planar electrode arrangement (2, 2') to which an electrical voltage can be supplied, and a planar shielding layer (1) consisting of an isolating plastic, which at least partially encloses the electrode arrangement (2, 2'), the electrode arrangement (2, 2') consisting of a plastic provided with conductive additives, **characterised in that** the in the region of the boundary layer (22) between the electrode arrangement (2, 2') and the shielding layer (1), the plastics of the electrode arrangement (2, 2') and the shielding layer (1) are connected to one another in material-bonding manner without an additional adhesive layer by the plastics being mixed and/or cross-linked with one another in the region of the boundary layer (22).

2. Treatment arrangement according to claim 1, **characterised in that** the electrode arrangement (2, 2') is enclosed on all sides by the shielding layer (1).

3. Treatment arrangement according to claim 1 or 2, **characterized in that** an electrically conductive terminal of the electrode arrangement (2, 2') is led out of the shielding layer (1).

4. Treatment arrangement according to one of claims 1 to 3, **characterised in that** a contact arrangement for supplying the electrical voltage can be led through the shielding layer (1) to the electrode arrangement (2, 2').

5. Treatment arrangement according to one of claims 1 to 4, **characterised in that** the shielding layer (1) is profiled on the treatment side (13) to form application surfaces (16'), between which air intermediate spaces (17) for forming the plasma exist upon application of the treatment arrangement to the surface to be treated.

6. Treatment arrangement according to one of claims 1 to 5, **characterised by** a wound dressing surface formed on the treatment side (13).

7. Treatment arrangement according to one of claims 1 to 6, **characterized in that** the plastics of the electrode arrangement (2, 2') and the shielding layer (1) are chemically identical.

8. Treatment arrangement according to one of claims 1 to 7, **characterised in that** the plastics of the electrode arrangement (2, 2') and the shielding layer (1) are silicones.

9. Treatment arrangement according to any one of claims 1 to 8, **characterized in that** the shielding layer (1) has sections (10) protruding beyond the surface of the electrode arrangement (2, 2'), which are formed as adhesive toward the surface to be treated.

10. Treatment arrangement according to one of claims 1 to 9, **characterized in that** the electrode arrangement (2, 2') is connectable to a pole of the electric voltage and is designed such that the surface to be treated can form a counter electrode.

11. Treatment arrangement according to one of claims 1 to 10, **characterized in that** the electrode arrangement (2) has two electrode strips (4), which are connectable to two voltage-conducting poles (8) of the electric voltage.

12. Treatment apparatus with a treatment arrangement according to one of claims 1 to 11, **characterized by** a high-voltage supply device (9), which is connected to the electrode arrangement (2, 2') to form a plasma between a planar treatment side (13) of the treatment arrangement and the surface to be treated.

13. Method for producing a treatment arrangement according to one of claims 1 to 12, **characterised in that,** at least in the region of a boundary surface (22) between electrode arrangement (2, 2') and shielding layer (1), the plastics are mixed with one another in a liquid state and jointly cured and/or crosslinked.

14. Method according to claim 13, **characterised in that** firstly at least one layer of the shielding layer (1), then plastic of the electrode arrangement (2, 2') provided with the conductive additives in the liquid state are introduced into a casting mold, so that mixing results in the boundary region (22) between the plastics and **in that** subsequently the plastics are jointly cured and/or crosslinked.

15. Process according to claim 13, **characterized in that** a first of the plastics is partially crosslinked, and **in that** subsequently a second of the plastics is conducted onto the partially crosslinked first plastic in the non-crosslinked state and a further crosslinking of the partially crosslinked first plastic is carried out with the crosslinking of the second plastic.

16. Process according to claim 13, **characterized in that** a first of the plastics has crosslinkable functional groups in the crosslinked state, and **in that** a materially-bonded connection is produced with a second of the plastics by a secondary crosslinking of the second plastic using the crosslinkable groups of the first plastic.

## Revendications

1. Ensemble de traitement pour traiter une surface, comprenant un ensemble d'électrode surfacique (2, 2') pouvant être alimentée en une tension électrique, et une couche de protection surfacique (1) constituée en une matière plastique isolante et entourant au moins partiellement ledit ensemble d'électrode (2, 2'), l'ensemble d'électrode (2, 2') étant constitué en une matière plastique pourvue d'additifs conducteurs,
**caractérisé en ce que**
au niveau de la couche limite (22) entre l'ensemble d'électrode (2, 2') et la couche de protection (1), les matières plastiques de l'ensemble d'électrode (2, 2') et de la couche de protection (1) sont reliées les unes aux autres par coopération de matière sans une couche de colle supplémentaire du fait que les matières plastiques sont mélangées et/ou réticulées les unes avec les autres au niveau de la couche limite (22).

2. Ensemble de traitement selon la revendication 1,
**caractérisé en ce que**
l'ensemble d'électrode (2, 2') est enfermé sur tous les côtés par la couche de protection (1).

3. Ensemble de traitement selon la revendication 1 ou 2,
**caractérisé en ce que**
une borne électriquement conductrice de l'ensemble d'électrode (2, 2') est menée hors de la couche de protection (1).

4. Ensemble de traitement selon l'une des revendications 1 à 3,
**caractérisé en ce que**
un ensemble de contact destiné à l'alimentation en tension électrique peut être mené à travers la couche de protection (1) vers l'ensemble d'électrode (2, 2').

5. Ensemble de traitement selon l'une des revendications 1 à 4,
**caractérisé en ce que**
la couche de protection (1) est profilée sur le côté traitement (13) pour réaliser des surfaces d'appui (16') entre lesquelles existent des interstices d'air (17) pour réaliser le plasma lors de l'appui de l'ensemble de traitement contre la surface à traiter.

6. Ensemble de traitement selon l'une des revendications 1 à 5,
**caractérisé par**
une surface de recouvrement de blessure réalisée sur le côté traitement (13).

7. Ensemble de traitement selon l'une des revendications 1 à 6,
**caractérisé en ce que**
les matières plastiques de l'ensemble d'électrode (2, 2') et de la couche de protection (1) sont chimiquement identiques.

8. Ensemble de traitement selon l'une des revendications 1 à 7,
**caractérisé en ce que**
les matières plastiques de l'ensemble d'électrode (2, 2') et de la couche de protection (1) sont des silicones.

9. Ensemble de traitement selon l'une des revendications 1 à 8,
**caractérisé en ce que**
la couche de protection (1) comprend des portions (10) qui font saillie au-dessus de la surface de l'ensemble d'électrode (2, 2') et qui sont réalisées de manière à coller vers la surface à traiter.

10. Ensemble de traitement selon l'une des revendications 1 à 9,
**caractérisé en ce que**
l'ensemble d'électrode (2, 2') peut être connecté à un pôle de la tension électrique et est réalisé de telle sorte que la surface à traiter peut former une contre-électrode.

11. Ensemble de traitement selon l'une des revendications 1 à 10,
**caractérisé en ce que**
l'ensemble d'électrode (2) comprend deux bandes d'électrode (4) qui peuvent être connectées à deux pôles sous tension (8) de la tension électrique.

12. Dispositif de traitement comportant un ensemble de traitement selon l'une des revendications 1 à 11,
**caractérisé par**
un système d'alimentation en haute tension (9) qui est connecté à l'ensemble d'électrode (2, 2') pour réaliser un plasma entre un côté traitement surfacique (13) de l'ensemble de traitement et la surface à traiter.

13. Procédé de réalisation d'un ensemble de traitement selon l'une des revendications 1 à 12,
**caractérisé en ce que**
au moins au niveau d'une surface limite (22) entre l'ensemble d'électrode (2, 2') et la couche de protection (1), les matières plastiques sont mélangées les unes avec les autres dans un état liquide et durcies et/ou réticulées conjointement.

14. Procédé selon la revendication 13,
**caractérisé en ce que**
tout d'abord au moins une strate de la couche de protection (1) et ensuite une matière plastique, pourvue des additifs conducteurs, de l'ensemble d'électrode (2, 2') sont introduites dans un moule de coulée, à l'état liquide, de sorte qu'un effet de mélange se produit dans la zone limite (22) entre les matières plastiques, et qu'ensuite les matières plastiques sont durcies et/ou réticulées conjointement.

15. Procédé selon la revendication 13,
**caractérisé en ce que**
une première des matières plastiques est réticulée partiellement, et ensuite une seconde des matières plastiques est amenée à l'état non réticulé sur la première matière plastique réticulée partiellement, et lors de la réticulation de la seconde matière plastique s'effectue une autre réticulation de la première matière plastique réticulée partiellement.

16. Procédé selon la revendication 13,
**caractérisé en ce que**
une première des matières plastiques présente à l'état réticulé des groupes fonctionnels réticulables, et **en ce que**
avec une seconde des matières plastiques, une liaison par coopération de matière est réalisée par une réticulation secondaire de la seconde matière plastique avec les groupes réticulables de la première matière plastique.
